# EUROPÄISCHE PATENTSCHRIFT

(11) **EP 1 432 439 B1**
(45) Veröffentlichungstag und Bekanntmachung des Hinweises auf die Patenterteilung: **07.12.2005**
(21) Anmeldenummer: 02781132.2
(22) Anmeldetag: 02.10.2002
(51) Int. Cl.: A61K 39/00, C12N 15/85, C12N 15/87

(54) **MITTEL ZUR VERBESSERUNG DER IMMUNANTWORT**
MEANS FOR IMPROVING IMMUNE RESPONSE
AGENT DESTINE A AMELIORER LA REPONSE IMMUNITAIRE

(30) Priorität: 02.10.2001 DE 10148697; 12.11.2001 DE 10156678
(43) Veröffentlichungstag der Anmeldung: 30.06.2004
(73) Patentinhaber: Mologen AG, 14195 Berlin (DE)
(72) Erfinder: MORENO LOPEZ, Sonia, 28004 Madrid (ES); TIMON JIMENEZ, Marcos, 28200 San Lorenzo de El Escorial (ES)
(74) Vertreter: Bergmann, Simon
(86) Internationale Anmeldenummer: PCT/DE2002/003798
(87) Internationale Veröffentlichungsnummer: WO 2003/031469

(56) Entgegenhaltungen:
- EP-A- 0 941 318
- SCHIRMBECK REINHOLD ET AL: "Priming of immune responses to hepatitis B surface antigen with minimal DNA expression constructs modified with a nuclear localization signal peptide." JOURNAL OF MOLECULAR MEDICINE (BERLIN), Bd. 79, Nr. 5-6, Juni 2001 (2001-06), Seiten 343-350, XP002252260 ISSN: 0946-2716 in der Anmeldung erwähnt
- MCCLUSKIE M J ET AL: "ROUTE AND METHOD OF DELIVERY OF DNA VACCINE INFLUENCE IMMUNE RESPONSES IN MICE AND NON-HUMAN PRIMATES" MOLECULAR MEDICINE, BLACKWELL SCIENCE, CAMBRIDGE, MA, US, Bd. 5, Nr. 5, Mai 1999 (1999-05), Seiten 287-300, XP002941186 ISSN: 1076-1551
- SHI NING ET AL: "Immune responses affected by different injection methods of a multi-epitope chimeric DNA vaccine of Plasmodium falciparum." ZHONGHUA WEISHENGWUXUE HE MIANYIXUE ZAZHI, Bd. 21, Nr. 1, Januar 2001 (2001-01), Seiten 50-54, XP001154098 ISSN: 0254-5101
- LOPEZ-FUERTES L ET AL: "DNA vaccination with linear minimalistic (MIDGE) vectors confers protection against Leishmania major infection in mice" VACCINE, BUTTERWORTH SCIENTIFIC. GUILDFORD, GB, Bd. 21, Nr. 3-4, 13. Dezember 2002 (2002-12-13), Seiten 247-257, XP004394510 ISSN: 0264-410X
- "Form Follows Function: Introduction to the MIDGE Vector Technology" INTERNET PUBLICATION, [Online] 31. Mai 2002 (2002-05-31), XP002252259 Gefunden im Internet: <URL:http://web.archive.org/web/2002060214 1807/www.midge.com/technology/index.html> [gefunden am 2003-08-21]

## Beschreibung

Die Erfindung betrifft die Verwendung eines in eukaryoten Zellen operablen DNA-Expressionskonstrukts zur Herstellung eines Impfstoffes zur intradermalen Injektion zur Erzeugung einer Typ 1 zellulär vermittelten Immunantwort, sowie ein entsprechendes Mittel zur Verbesserung der Immunantwort durch Kopplung von transfervermittelnden Molekülen an Genexpressionskonstrukte.

Die Erfindung betrifft das Gebiet der genetischen Immunisierung.

Die genetische Immunisierung beruht auf dem Prinzip, dass nicht wie herkömmlich geschwächte Erreger oder diese charakterisierende Antigene, sondern nur noch Genexpressionskonstrukte geimpft werden. Diese kodieren für immunogene Proteine viraler, bakterieller oder parasitärer Erreger oder bei malignen Erkrankungen spezifisch exprimierter bzw. präsentierter Antigene. Dem Impfling wird somit lediglich die genetische Information zur Herstellung des fremden Proteins verabreicht, worauf die Körperzellen das fremde Protein selbst herstellen und anschließend gegen dieses körperfremde Protein eine effiziente Immunantwort ausgebildet wird.

### Hintergrund der Erfindung

Der Schutz vor Infektionskrankheiten und auch das Prinzip der Immunisierung beruhen auf der Wiedererkennung von Strukturen von in der Vergangenheit erfolgreich bekämpften Erregern durch das Immunsystem.

Dabei werden zwei Hauptwege unterschieden: Das humorale, welches auf der Synthese von Antikörpern durch B-Lymphozyten aber auch humoralen Komponenten der nicht-adaptiven Immunität, dem Komplementsystem, beruht, und das zelluläre Immunsystem, welches auf der Aktivität von T-Lymphozyten, NK-Zellen und antigenpräsentierenden Zellen des Immunsystems beruht. T-Lymphozyten sind in der Lage, mit Viren infizierte Zellen zu erkennen. Man weiß heute, daß der zelluläre Arm des Immunsystems durch Aktivierung von sog. Typ- 1- Helferzellen, und der humorale Arm durch Aktivierung von sog. Typ-2-Helferzellen induziert wird (Mosmann et al., J. Immunol. 1986, 136(10): 3561-6). Entsprechend wird der zelluläre Arm auch als Th1 pathway und der humorale Arm als Th2 pathway bezeichnet. Extrazellulär vorliegende Bakterien werden in der Regel durch den Th2 Arm bekämpft. Der Th2 Arm ist ebenso wichtig für die Neutralisierung von Bakteriengiften und für die Bekämpfung verschiedener Parasiten, die sich im extrazellulären Raum im Körper befinden.

Infektionserreger, die sich vor allem intrazellulär aufhalten, wie es für einzelne Bakterienarten und alle Viren bekannt ist, werden dagegen hauptsächlich durch den Th1 Arm, also durch zytotoxische Zellen, bekämpft.

Zum Einschleusen "(Transfektion") der die immunogenen Antigene oder Teile davon kodierenden DNA in Zellkerne antigenpräsentierender oder sonstiger somatischer Zellen sind verschiedene chemische, physikalische und biologische Transfektionsmethoden bekannt.

Mittel zur Transfektion, sog. Genfähren, sind virale Vektoren, Plasmide oder kovalent geschlossene minimalistische DNA-Konstrukte (vgl. EP 0914 318 B1; im folgenden als MIDGE® (MINIMALISTIC IMMUNOLOGICALLY DEFINED GENE EXPRESSION VECTORS) bezeichnet). Wildtyp-Viren und eng mit ihnen verwandte Vektoren zeigen eine in der Regel hohe Transfektionseffizienz und gute Gewebespezifität, sind allerdings aufgrund von Sicherheitsbedenken und dem Problem der gegen den Vektor auftretenden Immunität umstritten. Diese letzteren Probleme treten bei der Verwendung von "nackter" DNA nicht auf. Bei der in vivo wie auch der in vitro Transfektion mit solchen in der Regel von Plasmiden abgeleiteten Systemen stellt sich das Problem der effizienten sowie zellart- oder gewebespezifischen Transfektion. Aus diesem Grunde wurde versucht, die auf DNA beruhenden Transfektionsmethoden zu optimieren. Verschiedene Peptide und andere organische Moleküle wurden an Genfähren über unterschiedliche Bindungsarten gekoppelt. Ebenso wurden durch Kopplung von Liganden, die Ligand-Rezeptor Wechselwirkungen zur verstärkten Aufnahme der Genfähren, ausgenutzt (Fraser et al., 1998, Semin. Immunl., 10 (5): 363-72).

Durch kovalente Kopplung des Kemlokalisationssignals (NLS) aus dem SV 40 Virus an für das Hepatitis small surface Antigen (HBsAg) kodierende Expressionskassetien konnte ein 10- bis 15-fach erhöhter Antikörpertiter nach intramuskulärer Applikation nachgewiesen werden (Schirmbeck et al., J. Mol Med. 2001 Jun;79 (5-6):343-50). Es wurde ein Unterschied in der Isotypenverteilung der Antikörperantwort gefunden, mit einer starken Übergewichtung des Th2-spezifischen IgG1-Subtyps nach intradermaler Applikation mit partikelgebundener DNA ("Gene-Gun"), und einem Übergewicht an Th1-typischem Subtyp IgG2a nach intramuskulärer Applikation. Es wurde kein Unterschied der Subtypenverteilung gefunden, der mit den Vektoren (Plasmid, minimalistischer Vektor) korrelierte.

Ebenso wurde das elf Aminosäuren lange T-Peptidfragment (YGRKKRRQRRR) des HIV-1 Genprodukts TAT verwendet, um 200nm große Liposomen in Zellen einzuschleusen. Die erfolgreichen Experimente verlangten jedoch eine Konzentration von rund 500 T-Peptiden pro Liposom (Torchilin et al., PNAS 2001, 98(15): 8786-8791). Der Nachweis, dass es eine Membran-durchdringende Funktion aufweist, welche den Transport von Proteinen oder Peptiden in Zellen unterstützt, gelang Frankel (Frankel et al., 1988, Cell. 55: 1179-1188, Green et al., 1988, Cell. 55: 1179-1188, US 5670617).

Es ist für die Impfung bzw. Immuntherapie verschiedener Krankheiten offenbar vorteilhaft, eine Th1-typische Immunreaktion zu erzeugen. Insbesondere gilt dies für intrazelluläre Parasiten wie Leishmania und Malaria, sowie virale Erkrankungen wie HIV. Jedes Mittel, welches die Ausprägung der Th1-Antwort nach Injektion von DNA verstärkt, ist in diesem Sinne vorteilhaft für die Erzeugung eines wirkungsvollen Impfschutzes.

Aufgabe der Erfindung ist es, ein Mittel zur Verfügung zu stellen, welches eine pro Menge eingebrachter DNA-Expressionskonstrukte stärkere sowie vermehrt zellulär vermittelte (Th1) Immunantwort erzeugt als die im Stand der Technik zur Verfügung stehenden Mittel.

Gelöst wird die Aufgabe durch die unabhängigen Ansprüche.

Es wurde gefunden, dass durch die kovalente Bindung eines Peptides mit einer Proteintransduktionsdomäne oder einer Kemlokalisationsdomäne an die Haamadelschleife von kovalent geschlossenen DNA Konstrukten (MIDGE), welche ein impfantigen kodieren, und anschließender impfung der Konstrukte in ein Tier, die Art und Stärke der resultierenden Immunantwort signifikant in Richtung einer zellulären Immunantwort verändert wird. Zusammenfassend schafft die vorliegende Erfindung daher ein Arzneimittel zur Erzeugung einer Typ1 zellulär vermittelten Immunantwort durch intradermale Injektion peptidkonjugierter DNA-Expressionskonstrukte zur Expression von Antigenen in Lösung. Die Erfindung hebt sich deutlich vom Stand der Technik unter anderem dadurch ab, dass die Induktion einer solchen Typ 1 Antwort vorher von den maßgeblichen wissenschaftlichen Fachkreisen gerade nicht erwartet worden war; hierfür sei als Beleg beispielhaft auf den Artikel von einem der Begründer der DNA-Impfung, David Weiner, im Joumal of Leukocyte Biology (Shedlock und Weiner, J. Leukocyte Biol. Vol 68, Dec. 2000, 793-806) hingewiesen: Die Autoren formulieren expilizit die Erwartungen an verschiedene Applikationsformen (ibid., S. 795 linke Spalte unten): *"Forms of delivery targeting the skin, including i.d. injection* ... *have been shown to elicit a humoral response primarily, characterized by a rapid progression to a Th2-type response, associated with the production of an IgA and IgG1 antibody isotype."* Eine Th1-Antwort war also gerade nicht erwartet worden, weswegen die vorliegende Erfindung umso überraschender und unerwartet für den maßgeblichen Fachmann ist.

Ebenso konnte eine Zusammenarbeit der Anmelderin mit einer Arbeitsgruppe der Universität Ulm (Schirmbeck et al, wie zitiert) keine veränderten Antikörperspiegel im Vergleich zu einer Behandlung mit nicht-modifizierten Vektoren bei der Anwendung durch Partikel-Bombardierung der Haut nachweisen. Es ist damit davon auszugehen, dass die erfindungsgemäßen vorteilhaften Wirkungen sich auf die Anwendung durch Injektion in Lösung in die Haut beschränken.

Dies allerdings ist als großer technischer Fortschritt anzusehen. Denn zum Einen ist die Anwendung von DNA-Expressionskonstrukten in der Haut von Vorteil, weil hier mehr für die sekundäre Wirkung der Konstrukte verantwortliche Zellen residieren und damit mit weniger DNA geimpft werden kann. Zum anderen ist die intramuskuläre Injektion schmerzhafter sowie, bei Nutztieren, mit der Entstehung von Impfmalen verbunden, was die kommerzielle Verwertung der geimpften Stelle als Fleisch verhindert und zur Wertminderung des geimpften Tieres führt.

Es wurde das überraschende und nicht zu erwartende Ergebnis gefunden, dass das transduzierende Sequenzelement des TAT Peptides ein Nukleinsäurekonstrukt transportieren kann, welches um Größenordungen größer ist als sein natürliches Substrat. Dieses Ergebnis war deswegen nicht zu erwarten, weil bisherige Untersuchungen nur mit wesentlich kleineren Molekülen einen Effekt zeigten. Dowdy et al. zeigten, dass die Fusion dieser Peptidsequenz an Proteinmoleküle ausreichend ist, um ein 120 kDalton großes Protein in Zellen zu transportieren (Dowdy et al. 2000, Trends Pharmacol. Sci., 21 (2): 45-8). Es sollte daher überprüft werden, ob das von TAT abgeleitete T-Peptid auch in der Lage ist, nochmals erheblich größere DNA-Expressionkonstrukte zu transduzieren. Diese Annahme ist schon wegen der Molekülgröße (für ein 1800 bp großes Expressionskonstrukt liegt diese bei ca. 1,2 MDalton) sowie die vollkommen andere Molekülform weder trivial noch naheliegend. Während Proteine und Peptide nämlich allgemein in globulärer Form vorliegen, sind nicht topologisch gespannte DNA-Moleküle in erster Näherung als lineare Moleküle zu betrachten. Hinzu kommt für die Betrachtung ihrer Verschiedenheit von Proteinen noch die Oberflächenladung durch die Phosphatgruppen, die DNA zu einem stark negativ geladenen Molekül machen.

Ferner wurde aus dem Simian Virus SV-40 eine Peptidsequenz charakterisiert, welche ein Kern-Lokalisierungssignal (Nuclear Localization Signal = NLS) darstellt. Das Vorhandensein solcher Signalsequenzen, die für den Import von Proteinen in den Zellkern notwendig sind, sind aus mehreren Organismen bekannt. Moleküle, die größer als 60 kDa sind, können nur mit Hilfe einer Kern-Signalsequenz in den Zellkern transportiert werden. Speziell für das SV-40-NLS wurde gezeigt, dass Proteine bis zu 465 kDa zum Zellkern gesteuert werden (Lanford et al. 1986, Cell 15; 46 (4): 575-82). Diese Fähigkeit des Peptids wurde hier durch Kopplung an DNA für die Verbesserung des Gentransfers genutzt. Die verwendete Peptidsequenz ist PKKKRKV.

Das zugrundeliegende Verfahren zur Herstellung derartiger Nukleinsäurekonstrukte zur Transkription von RNA-Molekülen in einer Zelle oder einem Zellverbund beruht auf der EP 0 941 318 B1, wobei das Nukleinsäurekonstrukt
- aus einem zirkulären Strang Desoxyribonukleinsäure mit einer teilweise zueinander komplementären, antiparallelen Basensequenz besteht, so dass ein hantelförmiges Konstrukt entsteht,
- wobei der zueinander komplementäre, antiparallele Basenabschnitt im wesentlichen aus einem Promotor, einem kodierendem Bereich und entweder einem Poly(A)-Additionssignal oder einer anderen RNAstabilisierenden Sequenz besteht,
- und der nicht komplementäre Basenabschnitt zwei Schlaufen ('Haarnadelschleifen') einzelsträngiger Desoxyribonukleinsäure bildet, die jeweils das 5'- und 3'-Ende des komplementären, antiparallelen Basenabschnitts kovalent miteinander verbinden,
besteht, wobei
- mit Hilfe wenigstens eines der folgenden Oligonukleotide (ODN 1 oder ODN 2) wobei X ein Aminogruppen (NH₂)-modifiziertes aktiviertes Nukleosid (Thymin) kennzeichnet,
   die Haarnadelschleife ausgebildet wird,
- und mittels eines Crosslinkers wenigstens eine organische Molekülverbindung kovalent mit dieser Haamadelschleife verbunden wird.

Dieses Verfahren ermöglicht die kovalente Bindung von Molekülen an die Nukleinsäurekonstrukte. In aus dem Stand der Technik bekannten Problemlösungen erfolgt die Bindung der Liganden nicht direkt an das Plasmid, sondern über ein Brückenmolekül und die Bindungsposition ist nicht molekular definiert. Daher besteht die Gefahr, mit diesen Modifikationen die Funktionsfähigkeit des Promotors oder der therapeutischen Gene zu beeinfächtigen. Die molekular definierte Kopplung ermöglicht auch eine Charakterisierung der Konstrukte gemäss den Anforderungen moderner Arzneimittel-Herstellungsvorschriften, die die Verwendung solcher Vektoren als Arzneimittel erst möglich macht. Die Anmelderin hat in eigenen Experimenten gefunden, dass der beobachtete Effekt der Induktion einer TH1-Antowort bei nichtkovalenter Beimischung der Peptide nicht gefunden wird. Die kovalente Bindung ist aus den oben beschriebenen Gründen deutlich bevorteilt; Grund hierfür dürfte die Erhöhung der Effektivität der Transfektion und damit auch letztlich der Transkription aufgrund der starken kovalenten Bindung sein. Der beobachtete Effekt lässt sich nach Beobachtungen der Anmelderin durch Kopplung einer Reihe weiterer basischer Peptide erzielen, so dass die Erfindung nicht auf die Verwendung der beiden beschriebenen Peptidsequenzen beschränkt ist.

In vivo Versuche in Mäusen mit für HBsAg kodierende MIDGE Vektoren, sowie Impfversuche gegen Leishmania major mit dem p36 LACK Antigen, bestätigten die theoretischen Überlegungen. Erfindungsgemäß waren an die Vektoren verschiedene Peptide kovalent gekoppelt. Ein effektiver und dabei sicherer Impfschutz wurde durch den Vergleich verschiedener Impfregime ermittelt. Parameter waren die Stärke des Th2/Th1 shifts und der erreichte Immunschutz, der anhand der Größe der Läsionen nach der Belastungsinfektion mit Leishmania major Promastigoten ermittelt wurde. Dies ist insofern von Bedeutung, als das Leishmania-Maus-System Modellcharakter für die Th1-Th2 Dichotomie hat und hier neben den für jedes Antigen messbaren Surrogatparametern der Antikörpersubtypenmenge auch der erwünschte biologische Erfolg - Schutz vor Infektion - im Gesamtorganismus nachgewiesen werden konnte. Dies verdeutlicht auch den großen erfinderischen Schritt von der Verwendung "nackter" nicht peptidmodifizierter DNA zu den mit kationischen Peptiden gekoppelten Expressionskonstrukten. Während erstere keinen nennenswerten Schutz vor Testinfektion gewährten, konnte durch die zweimalige Gabe von peptidmodifizierten Expressionskonstrukten ohne jede weitere Zugabe von Interleukinen oder anderen Immunmodulatoren ein vollkommener Schutz gewährt werden. Dies deutet auf eine massive, durch die Messung von Surrogatparametem nicht ohne weiteres erfassbare, Umlenkung der Immunantwort auf eine Interferon gammagetragene Typ 1 Antwort (Th1 Immunantwort mit Interferon gamma-Zytokinprofil und Immunglobulin Th1 Subtypenübergewicht) durch das erfindungsgemäße lmpfregime hin. Die erfindungsgemäße Vakzine wird als Lösung verwendet.

Während der Präsentation von Antigen durch Antigen-Präsentierende Zellen (APC) aus naiven T-Helferzellen kommt es bereits zu einer Prägung der T-Helferzellen in Richtung auf eine Th1 (zytotoxische) oder Th2 (humorale) Immunantwort. Bestimmend für diese Prägung ist u.a. das Zytokinmillieu, in dem die Interaktion zwischen APC und Helferzelle stattfindet, sowie die Art der an der Interaktion beteiligten Rezeptoren (Pulendran et al., Science 193, 253-256, 2001).

Die Isotypenverteilung von Immunglobin gamma (IgG) gegen beide Antigene (HbsAg und p36/LACK) wurde bestimmt, da die Isotypen IgG1 und IgG2a die Ausprägung der gesamten Immunantwort widerspiegeln. Dabei sind IgG1 Subtypen charakteristisch für eine humorale Antwort, begleitet von einer verstärkten Ausschüttung von Interleukinen IL-4 und IL-10 durch aktivierte Lymphozyten; ein erhöhter Spiegel von Subtyp IgG2a ist typisch für eine zelluläre Th1-Antwort, begleitet von erhöhter Ausschüttung von IFNγ und IL-12. Das Auftreten der Isotypen ist dabei nicht exklusiv, die relativen Titer können jedoch als Indikator für die dominante Art der entstandenen Immunantwort gewertet werden.

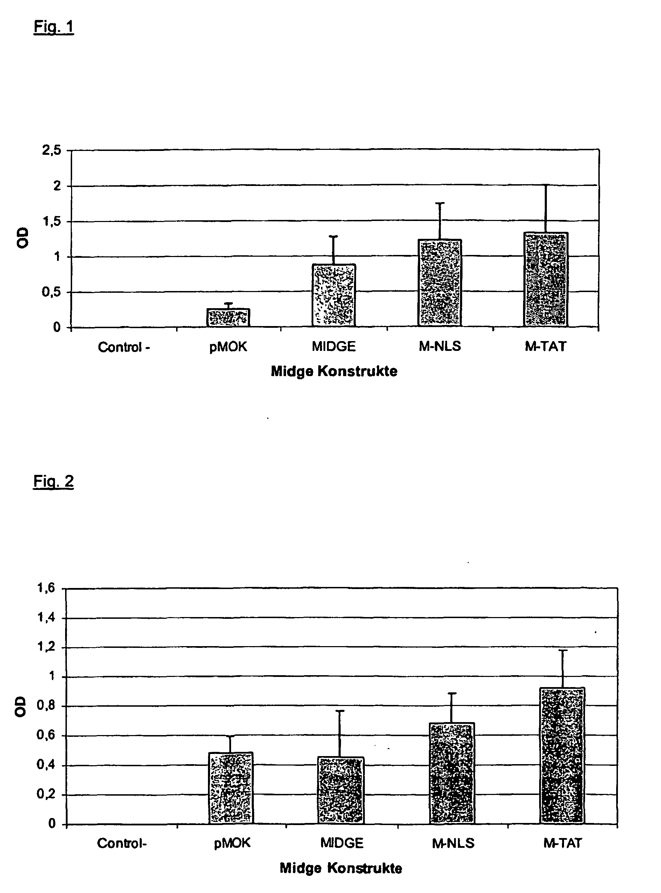

Die intradermale Applikation von HBsAg-kodierenden Plasmiden in Lösung führte nur zu einer Th2 - typischen Immunantwort (siehe Fig. 1). Eine Th1 typische Verschiebung der Antikörperisotypen durch Plasmid fand nicht statt (s. Fig. 3). Für eine Reihe medizinisch sehr relevanter Krankheiten ist allerdings die Erzeugung einer zytotoxischen Antwort wesentlich, hierzu gehören u.a. die Hepatiden, leukivirale Infektionen wie HIV sowie Infektionen mit intrazellulären Parasiten. Insofern ist die erfindungsgemäße Erzeugung einer Th1-dominanten Immunantwort durch Peptidgekoppelte Genexpressionsvektoren nicht nur eine quantitative Verbesserung, weil mit weniger DNA ein höherer Titer erreicht wird. Vielmehr handelt es sich um eine qualitative Verbesserung gegenüber dem Stand der Technik, die nach den aus der Literatur bekannten Daten keineswegs zu erwarten gewesen wäre.

Der hinter dieser qualitativen Verschiebung stehende Mechanismus ist dabei z.Z. nicht geklärt. Die Tatsache, dass die Ligandenkopplung an die minimalistischen Expressionsvektoren zu einer Erhöhung der Reporter-Genexpression in-vitro führt (Ergebnisse nicht gezeigt), sagt nicht unbedingt eine erhöhte Immunisierung voraus, noch weniger deren T_{H}- Subtyp. Eine Verringerung der applizierten DNA-Menge soiite, wenn überhaupt eine Vorhersage getroffen werden könnte, nach derzeitigem Kenntnisstand durch die Verringerung der Adjuvanz-Wirkung der zugeführten bakteriellen immunstimmulatorischen DNA-Motive zu einer Th2 - Antwort führen. Bei den untersuchten Vektoren mit NLS-und T-Peptid ist das Gegenteil der Fall

Die Ergebnisse des Impfversuchs gegen das p36 LACK Antigen von L. major zeigen, dass das erfindungsgemäße Mittel in seiner Schutzwirkung das im Stand der Technik als derzeit "bestes" bezeichnete Impfregime der Zweitimmunisierung (Boost) mit rekombinantem Vaccinia Virus (rW) übertrifft und vermeidet zusätzlich die möglichen Nebenwirkungen, die von Plasmiden und attenuierten Viren ausgehen, und ist doch gleichzeitig vergleichbar in seiner Schutzwirkung (Gonzalo et al., Microbes and Infection:3 (9) :701-711). Bei gleicher bzw. besserer Schutzwirkung ist das erfindungsgemäße Mittel einfacher, billiger und vor allem sicherer herzustellen.

Die Ergebnisse für die in vivo Versuche mit HBsAg sind sehr überraschend, da diese mit einer erheblich geringeren DNA-Menge erzielt werden konnten, als bisher für die Immunisierung mit nicht adjuvantierter DNA ohne partikuläre Formulierung in der Literatur beschrieben war. Für die Immunisierung mit HBsAg geht man allgemein von einer Menge von 30 - 100 µg Plasmid bei intramuskulärer oder intradermaler Injektion aus (Schirmbeck et al., 1998, Vaccine. Vol.16, No.9/10: 949-954).

Die Vorteile der Erfindung lassen sich mithin wie folgt zusammenfassen.
- Minimalistische Nukleinsäurekonstrukte werden so modifiziert, dass die Immunantwort verbessert wird.
- diese modifizierten minimalistischen Nukleinsäurekonstrukte führen eine verstärkt zellulär vermittelte Immunantwort herbei.

Die überraschende Wirkung der Verwendung des erfindungsgemäßen DNA-Expressionskonstrukts und einer Vakzine, die ein solches enthält, wird anhand der Darstellungen gem. der Figuren deutlich. Dabei bedeuten:
- pMOK p36: Plasmid kodierend für p36 Antigen
- Mp36-NLS: MIDGE kodierend für p36 Antigen mit NLS Peptid gekoppelt
- pMOK ctr: Kontrollplasmid kodierend für HBsAg
- rWp36: rekombinanter Vaccinia Virus kodierend für p36
- Phosphat: Phosphatpuffer als Kontrolle
- Kontrolle +: Positivkontrolle, mit L. major infizierte Seren von Mäusen
- Kontrolle -: Negativkontrolle, Seren unbehandelter Mäuse

Weitere vorteilhafte Maßnahmen sind in den übrigen Unteransprüchen beschrieben; die Erfindung wird nachfolgend anhand von Ausführungsbeispielen und Figuren näher beschrieben. Es zeigt
- **Fig. 1**: ELISA zur Bestimmung des Gesamt-IgG gegen Hepatitis small surface Antigen (HBsAg) in Mäusen;
- **Fig. 2**: ELISA zur Bestimmung des anti-HBsAg Gesamt-IgG nach Boost mit 1µg DNA;
- **Fig. 3 und 4**: Bestimmung der anti-HBsAg IgG Isotypen IgG 1 und IgG 2a;
- **Fig. 5**: Ergebnisse eines Impfversuchs gegen das p36 LACK Antigen von Leishmania major.
- **Fig. 6**: Bestimmung des Gesamt IgG Antikörpertiter gegen das p36 LACK Antigen nach der Belastungsinfektion mit Leishmania major. Alle Impfprotokolle zeigen eine messbare Antikörperantwort, wobei der höchste Titer an zirkulierenden Antikörpern von MIDGZ p36-NLS / MIDGE p38-NLS augelöst wird.

### Ausführungsbeispiele

### 1. Aktivierung der Oligodesoxyribonukleotide (ODN) zur Kopplung von Thiolfunktionalisierten Molekülen

Die Kopplung von Molekülen wie Peptiden, Zuckern oder anderen Naturstoffen ist generell über eine Vielzahl von chemischen Reaktionen denkbar. Es sind Standardreaktionen zur Herstellung von Amid-, Ester- oder Imidbindungen aus dem Repertoire der organischen Synthesechemie hinreichend bekannt. Zur konzeptionellen Überprüfung wurde hier die Reaktion einer Thiolgruppe, die am zu koppelnden Molekül vorhanden war, mit einer Maleinsäureimidgruppe am Nukleinsäuremolekül verwendet. Die Maleinimidgruppe wurde über die Reaktion eines bei der Synthese des ODN eingefügten Aminorests (mit X gekennzeichnet) mit einem kommerziell erhältlichen bifunktionellen Kopplungsreagenz über eine Transamidierung des im Kopplungsreagenz vorliegenden NHS-Carbonsäurederivats in die Nukleinsäurekomponente der Reaktion eingeführt. Zur Herstellung von einfach modifizierten MIDGE-Konstrukten wurde das in der Haarnadelschlaufe mit einem aminogruppenmodifizierten desoxy-Uracil (XT) versehene ODN 1 sowie das nichtmodifizierte ODN 2 verwendet:
5'-PH-GGG AGT CCA GT XT TTC TGG AC (TIB-Molbiol, Berlin, Kurzbezeichnung: ODN 1 = Seq ID 5), wobei PH für am 5'-OH phosporyliert steht.
und
5'-PH-AGG GGT CCA GTT TTC TGG AC (TIB-Molbiol, Berlin, Kurzbezeichnung: ODN 2 = Seq ID 6).

Das aminomodifizierte ODN 1 wurde wie folgt zur Kopplung eingesetzt: Der Crosslinker zur kovalenten Bindung (hier: sulfo-KMUS (N-(Maleimidoundecanoyloxy) sulfosuccinimid) in DMF, PIERCE Produkt-Nr. 21111) wurde in vier gleichen Teilen im Abstand von 30 min zu den Amino-ODN (0,1mM Endkonzentration) gegeben, bis eine Endkonzentration von 5 mM erreicht war. Die Reaktion erfolgte für zwei Stunden in einem Crosslink-Kopplungspuffer (50mM NaHPO₄, 75mM NaCl, pH 7,6) bei 37°C. Anschließend wurde die Reaktion durch Zugabe von Tris-HCl (pH 7,5; 50 mM Endkonzentration) gestoppt. Die aktivierten Amino-ODN wurden für 30 min bei -70 C Ethanol-präzipitiert (300 mM NaOAc pH 5,3; 20 mM MgCl₂; 2,5-faches Reaktionsvolumen 100% Ethanol abs.). Das Präzipitat wurde 30 min bei 15.000 rpm (4 C) zentrifugiert und unter gleichen Bedingungen für 15 min mit 70 % Ethanol gewaschen. Die aktivierten Amino-ODN wurden abschließend in Wasser (MilliQ-Qualität) aufgenommen und bis zur Kopplung bei -20 C eingefroren.

### 2. Kopplung von T- Peptid an aktivierte Oligodesoxyribonukleotide (ODN)

Die unter 1. beschriebenen aktivierten Amino-ODN wurden in Kopplungspuffer (1x = 50 mM NaHPO₄, 75 mM NaCl, pH 7,0) aufgenommen, so dass sich eine 0,1 millimolare Endkonzentration ergab. Anschließend wurde das in Wasser gelöste T-Peptid mit der Sequenz YGRKKRRQRRR (= Seq ID 3; hergestellt und freundlicherweise zur Verfügung gestellt von Dr. Peter Henklein, Charite, Berlin) in 0,2 mM Endkonzentration zugegeben. Die Reaktion erfolgte bei 37 °C für eine Stunde.

Die Reinigung und Trennung der resultierenden Peptid-gekoppelten ODN von den nicht gekoppelten ÖDN erfolgte mittels Reversed Phase-HPLC. Die einzelnen Reaktionen wurden gelelektrophoretisch analysiert. Die HPLC-Fraktion mit den T-ODN wurde in einer Vakuumzentrifuge eingeengt und in MilliQ-Wasser aufgenommen. Die modifizierten ODN wurden über eine Nukleosil-300 C18 Säule (10µm, 250mm Länge x 8mm Innendurchmesser) HPLC gereinigt. Der Gradient verlief von 0% Puffer A (100mM Ammoniumcarbonat) auf 42% Puffer B (80% Acetonitril) in 47min bei einer Flussrate von 2,4ml/min.

### 3. Kopplung von SV-40 NLS Peptid an aktivierte ODN

Die unter 1. beschriebenen aktivierten Amino-ODN wurden in Kopplungspuffer (1x = 50 mM NaHPO₄, 75 mM NaCl, pH 7,0) aufgenommen, so dass sich eine 0,1 mM Endkonzentration ergab. Anschließend wurde das in Wasser gelöste Peptid SV-40 NLS mit der Sequenz PKKKRKV (= Seq ID 4; Dr. Henklein) in 0,2mM Endkonzentration zugegeben. Die Reaktion erfolgte bei 37°C für eine Stunde.

Die Reinigung und Trennung der resultierenden Peptid-gekoppelten ODN von den nicht gekoppelten ODN erfolgte mittels Reversed Phase-HPLC. Die einzelnen Reaktionen wurden gelelektrophoretisch analysiert. Die HPLC-Fraktion mit den NLS-ODN wurde in einer Vakuumzentrifuge eingeengt und in MilliQ-Wasser aufgenommen.

### 4. Herstellung von MIDGE - HBsAg T-Peptid

MIDGE sind minimalistische Expressionsvektoren aus doppelsträngiger DNA, die nur aus der Expressionskassettte, d.h. aus dem CMV Promotor, einem Intron, der entsprechenden Gensequenz und einer Polyadenylierungssequenz bestehen. Die Konstrukte wurden wie folgt erhalten: Das Plasmid pMOK HBsAg wurde mit Eco 311 vollständig verdaut. Die Ligation mit 5' phosphorylierten haarnadelförmigen ODN 1, an das das T-Peptid entsprechend 1. gekoppelt war, und ODN 2 erfolgte durch T4 DNA Ligase in Anwesenheit von Eco 31I und wurde durch Erhitzen auf 70°C gestoppt. Das resultierende Gemisch wurde konzentriert und mit Eco 31I und T7 Polymerase in Abwesenheit von Desoxyribonukleotid-Triphosphaten behandelt. Die Aufreinigung erfolgte durch Anionenaustauschchromatographie. Der Nachweis der erfolgreichen Kopplung der verschiedenen Liganden an die MIDGE erfolgte durch Restriktionsverdau. Durch gelelektrophoretischen Größenvergleich der vom MIDGE Vektor (mit BamHI bzw. EcoRI) abgeschnittenen ODN mit entsprechenden Kontroll-ODN wurde bestätigt, dass die Vektoren mit Peptiden gekoppelt waren. (Die Sequenz HbsAg ist in Seq. ID 1 wiedergegeben)

Die Herstellung von MIDGE - HBsAg NLS-Peptid erfolgte entsprechend.

### 5. Klonierung des Plasmids pMOKp36

Vom Ausgangsplasmid pSCp36 wurden 2 Fragmente mittels PCR amplifiziert:
1. PCR ca. 800 bp;
2. PCR ca. 950 bp;

Das PCR-Produkt aus der 2. PCR wurde mit Eco31I geschnitten und das kleinere Fragment (ca. 200 bp) isoliert.

Das PCR-Produkt aus der 1. PCR wurde mit Bpil geschnitten.

Das 200 bp Fragment und das geschnittene Fragment aus der 1. PCR wurden zusamenligiert und anschließend mit Kpnl und Sacl geschnitten und in den mit Kpnl und Sacl geschnittenen pMOK-Vektor ligiert. Das entstandene Plasmid erhielt den Namen pMOK p36. Daraus wurde MIDGE p36-NLS hergestellt. (Die Sequenz p36 LACK ist in Seq. ID 2 wiedergegeben)

Die Aktivierung der ODN, die Kopplung der NLS Sequenz an die Oligonukleotide und die Herstellung der MIDGE p36-NLS erfolgte wie zuvor beschrieben.

### Ergebnisse

Die Ergebnisse werden anhand der Figuren im Detail nachfolgend beschrieben:

Fig. 1 zeigt die Bestimmung des Gesamt IgG HBsAg Titers. Der Titer wurde mittels ELISA Verfahren bestimmt, wobei die Absorption in OD (Optische Dichte) bei einer Wellenlänge von λ = 450 nm bestimmt wurde. Als Vergleich wurden Plasmid und unmodifizierter MIDGE Vektor eingesetzt. Die MIDGE mit den verschiedenen Kopplungen zeigten eine deutliche Erhöhung des Antikörpertiters, der auf eine verstärkte Expression des HBsAg hinweißt. Dabei bedeutet:
- pMOK:: Plasmid.
- MIDGE:: unmodifizierter MIDGE
- M-NLS:: MIDGE-NLS gekoppelt
- M-TAT:: MIDGE mit T-Peptid gekoppelt

Fig. 2 zeigt den Boosteffekt der Zweitimmunisierung mit 1µg DNA nach 11 Wochen. Die bei der Erst- und auch bei der Zweitimmunisierung eingesetzte DNA-Menge betrug 1µg DNA. Auch hier zeigten die modifizierten MIDGE einen die erzeugte Immunantwort erheblich verstärkenden Effekt.

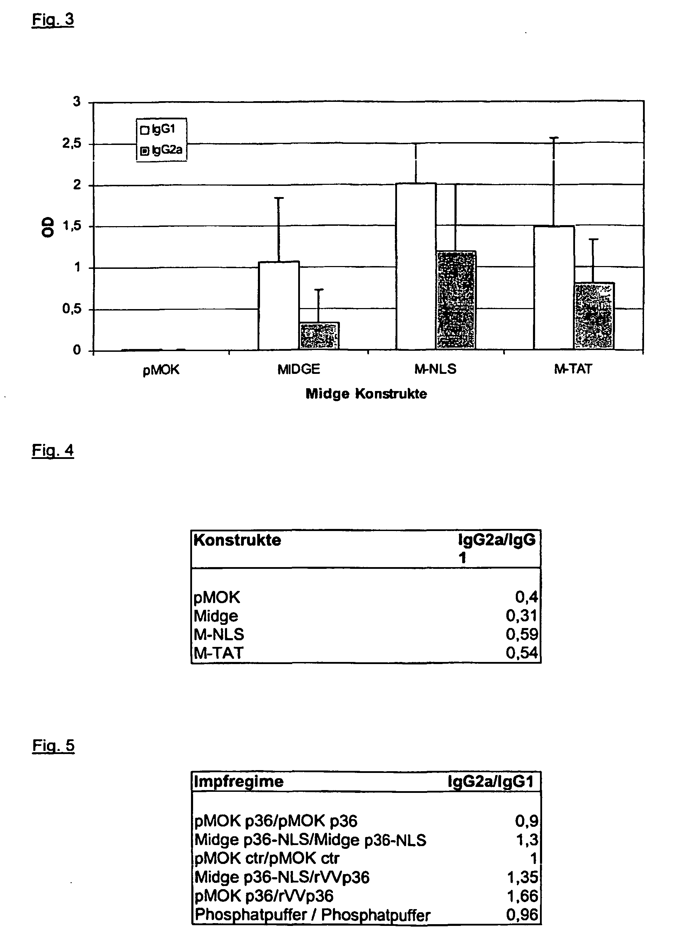

Fig. 3 und 4 zeigen die Bestimmung der IgG Isotypen IgG 1 und IgG 2a gegen HBsAg. Überraschenderweise lösten mit dem T-Peptid und mit der NLS-Sequenz gekoppelte MIDGE eine nach der Antikörper-isotypenverteilung zytotoxische Immunantwort (Th1) aus.

Fig. 5 zeigt das Verhältnis der Antikörper-Isotypen Verteilung IgG 2a und IgG 1 nach Zweitimmunisierung und Belastungsinfektion mit Leishmania major Promastigoten. Das Impfregime MIDGE p36-NLS/MIDGE p36-NLS zeigt den unerwarteten Effekt der Auslösung einer zellulären (Th1) Immunantwort. Der durch das Regime pMOKp36/rWp36 ausgelöste Th2/Th1 shift in der Immunantwort unterscheidet sich nur gering.

### Beispiel 1: HBsAg Antikörpertiterbestimmung in Mäusen

Gemäß Beispiel 4 wurden für das Hepatitis-B-Oberflächenantigen HBsAg (Subtyp ay) kodierende MIDGE hergestellt. Der Nachweis der Expression erfolgte durch Antikörpertiterbestimmung gegen das Hepatitis B Antigen mittels ELISA. Die MIDGE-Herstellung erfolgte entsprechend Ausführungsbeispiel 4. Im Einzelnen wurden unmodifizierte MIDGE und MIDGE mit einem Liganden, nämlich Migde-NLS und MIDGE-T hergestellt. Als zusätzliche Kontrolle wurde das Plasmid pMOK HBsAg eingesetzt. Als Negativkontrolle wurden die Seren unbehandelter Mäuse benutzt.

MIDGE (unmodifiziert sowie NLS-modifiziert) und Plasmid wurden gelöst in Natriumphosphat pH 7,2 in einem Volumen von 50 µl intradermal in Balb/c Mäuse injiziert. Die DNA- Mengen betrugen 10 µg bzw. 1µg pro Tier pro Impfung. Pro Gruppe wurden 5 Mäuse verwendet. Nach 11 Wochen erfolgte eine Zweitimmunisierung (Boost). Die Antikörpertiterbestimmung aus den Seren erfolgte nach Woche 2, 4 und 8. Die Ergebnisse sind in Fig. 1 dargestellt. Bei eingesetzten 10 µg DNA zeigt sich in der vierten Woche ein deutlicher Anstieg des Gesamt-Immunglobulin-G (lgG) Titers und damit die verstärkte Expression von HBsAg durch alle MIDGE Konstrukte gegenüber Plasmid. Den größten Effekt bewirkten die modifizierten MIDGE. Die Fehlerbalken stellen die Standardabweichung dar. Eingesetzte 1µg DNA führte zunächst zu keiner nennenswerten Steigerung des HBsAg Titers in Woche 4 (Ergebnisse nicht gezeigt); jedoch zeigte sich überraschenderweise ein starker Anstieg des Titers nach dem Boost mit 1µg in Woche 11. Wiederum zeigten die modifizierten MIDGE den stärksten Effekt (siehe Fig. 2). Diese Ergebnisse zeigen, dass auch mit minimaler DNA-Menge durch MIDGE ein hoher HBsAg-Antikörpertiter erreicht wird.

### Beispiel 2: Impfversuch gegen das Leishmania p36 Antigen

Zur Erzeugung eines effektiven Impfschutz gegen Leishmania major wurde das 36 kDa, auch als LACK, bezeichnete Antigen eingesetzt. In einem Impfversuch wurden verschiedene, für das immunogene p36 kodierende, Genfähren benutzt: MIDGE mit NLS Kopplung, Plasmid pMOKp36 und rekombinanter Vaccinia Virus p36 (rW). Um einen Vergleich für das wirksamste prime/boost Regime zu erhalten, wurden die Konstrukte nach folgendem Impfregime in weibliche (Balb/c) Mäuse injiziert.

| Gruppen | Erstimmunisierung (prime) | Zweitimmunisierung (boost) |
|---|---|---|
| 1 | pMOKp36 | pMOKp36 |
| 2 | MIDGE p36-NLS | MIDGE p36-NLS |
| 3 | pMOK Kontrolle | pMOK Kontrolle |
| 4 | pMOKp36 | rWp36 |
| 5 | MIDGE p36-NLS | rWp36 |
| 7 | Phosphatpuffer | Phosphatpuffer |

Je Gruppe wurden 10 Mäuse eingesetzt.
Die verwendeten DNA Mengen betrugen für:
pMOKp36: 100µg, i.d.
MIDGE p36-NLS: 54,8µg, i.d.
rVV p36: 5×10⁷ pfu/Maus, i.p.
und wurden in Natriumphosphatpuffer pH 7,2 gelöst, verabreicht.
Nach 2 Wochen erfolgte die 2. Impfung (boost) mit den entsprechenden (s. Tab.) DNA Konstrukten. Drei Wochen nach dem Boost erfolgte die Belastungsinfektion mit 5x10⁴ Leishmania major Promastigoten. Diese wurden den Mäusen in die rechte Hinterpfote s.c. injiziert.

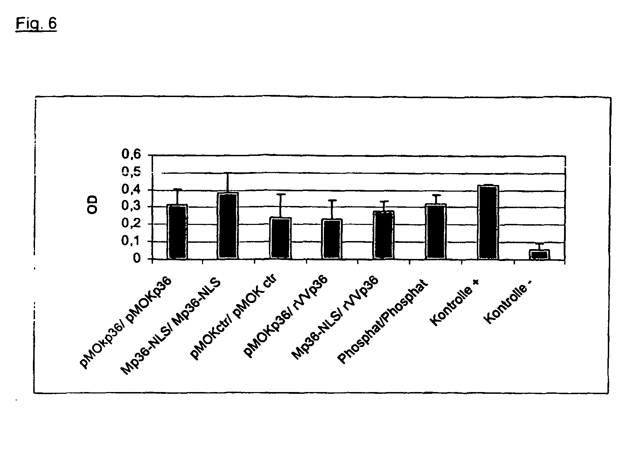

Acht Wochen nach der Belastungsinfektion wurden von allen Mäusen die Seren gewonnen (siehe Figur 6). Der Gesamt IgG Antikörpertiter gegen das p36 Antigen und die Bestimmung der IgG 2a und IgG 1 Antikörper erfolgte mittels ELISA, wobei die Absorption in OD (Optische Dichte) bei einer Wellenlänge von λ = 406 nm bestimmt wurde.

## Patentansprüche

1. Verwendung eines in eukaryoten Zellen operablen DNA-Expressionskonstrukts zur Herstellung eines Impfstoffes zur intradermalen Injektion zur Erzeugung einer Typ 1 zellulär vermittelten Immunantwort zum Schütz vor Infektionskrankheiten die durch intrazellulär Infektionserreger verursacht werden, wobei es sich bei dem DNA-Expressionskonstrukt um ein kovalent geschlossenes lineares Desoxyribonukleinsäuremolekül handelt, welches einen linearen Doppelstrangbereich aufweist, bei dem die doppelstrangbildenden Einzelstränge durch kurze einzelsträngige Schlaufen aus Desoxyribonukleinsäurenukleotiden miteinander verknüpft sind und die doppelstrangbildenden Einzelstränge nur aus de für ein oder mehrere Antigene kodierenden Sequenz unter Kontrolle eines im Impfling ope-rablen Promotors und einer Terminatorsequenz bestehen und ein solches DNA-Expressionskonstrukt zur Steigerung der Transfektionseffizienz mit einem oder mehreren Oligopeptiden kovalent verbunden ist.

2. Verwendung des DNA-Expressionskonstrukts nach Anspruch 1, wobei dieses für das Hepatitis small surface Antigen (HBsAg) kodiert.

3. Verwendung des DNA-Expressionskonstrukts nach Anspruch 1 oder 2, wobei das Oligopeptid eine Länge von fünf bis 25 Aminosäuren hat und mindestens die Hälfte der Aminosäuren zu der Gruppe Lysin oder Arginin gehören.

4. Verwendung des DNA-Expressionskonstrukts nach Anspruch 3, wobei das Oligopeptid eine Kernlokalisationssequenz trägt.

5. Verwendung des DNA-Expressionskonstrukts nach den Ansprüchen 3 oder 4, wobei das Oligopeptid die Sequenz PKKKRKV (Prolin-Lysin-Lysin-Lysin-Arginin-Lysin-Valin) enthält.

6. Verwendung des DNA-Expressionskonstrukts nach den Ansprüchen 3 oder 4, wobei das Oligopeptid die Sequenz YGRKKRRQRRR enthält.

## Claims

1. Use of an in eukaryotic cells operable DNA expression construct for the production of a vaccine for intradermal injection for the induction of a type-1 cellular mediated immune response for the protection against infection diseases caused by intracellular infection germs, where the DNA expression construct consists of a covalent closed dumbbell shaped desoxyribonucleic acid molecule, which has a linear double stranded part, where the double strand forming single strands are connected by short single stranded loops of desoxyribonucleic acid nucleotides and the double strand forming single strands consist only of a coding sequence for one or more antigens under the control of an in the vaccinated host operable promoter and a termination sequence and such a DNA expression construct is covalently connected with one ore more oligopeptids for the improvement of the transfection efficiency.

2. Use of a DNA expression construct according to claim 1, where it codes for the hepatitis small surface antigen (HBsAg).

3. Use of a DNA expression construct according to claim 1 or 2, where the oligopeptid has a length of five to 25 amino acids and at least half of the amino acids belong to the group of lysine and arginine.

4. Use of a DNA expression construct according to claim 3, where the oligopeptid carries a nuclear localisation sequence.

5. Use of a DNA expression construct according to claim 3 or 4, where the oligopeptid contains the sequence PKKKRKV (proline-lysine-lysine-lysine-arginine-lysine-valin).

6. Use of a DNA expression construct according to claim 3 or 4, where the oligopeptid contains the sequence YGRKKRRQRRR.

## Revendications

1. Utilisation d'un construct d'expression d'ADN apte à fonctionner dans les cellules eucaryotes destiné à la production d'un vaccin pour injection intradermique dans le but de générer une réponse immunitaire de type 1 transmise par voie cellulaire afin de protéger contre des maladies infectieuses provoquées par des agents infectieux intracellulaires, le construct d'expression d'ADN étant une molécule d'acide désoxyribonucléique linéaire fermée de façon covalente qui comporte une région bicaténaire linéaire dans laquelle les simples brins constituant le double brin sont reliés entre eux par de courtes boucles monocaténaires de nucléotides d'acide désoxyribonucléique et les simples brins constituant le double brin ne sont composés que de la séquence codant pour un ou plusieurs antigène(s) sous contrôle d'un promoteur apte à fonctionner dans un organisme ou sujet à vacciner et d'une séquence terminateur, un tel construct d'expression d'ADN étant relié de manière covalente à un ou plusieurs oligopeptide(s) pour augmenter l'efficacité de transfection.

2. Utilisation du construct d'expression d'ADN selon la revendication 1, celui-ci codant pour l'antigène de petite surface de l'hépatite B (HBsAg).

3. Utilisation du construct d'expression d'ADN selon la revendication 1 ou 2, l'oligopeptide ayant une longueur de 5 à 25 acides aminés et au moins la moitié des acides aminés appartenant au groupe de la lysine ou de l'arginine.

4. Utilisation du construct d'expression d'ADN selon la revendication 3, l'oligopeptide étant porteur d'une séquence de localisation nucléaire.

5. Utilisation du construct d'expression d'ADN selon la revendication 3 ou 4, l'oligopeptide contenant la séquence PKKKRKV (Proline-Lysine-Lysine-Lysine-Argini ne-Lysine-Valine).

6. Utilisation du construct d'expression d'ADN selon la revendication 3 ou 4, l'oligopeptide contenant la séquence YGRKKRRQRRR


### SEQUENCE LISTING

<110> Mologen Forschuungs-, Entwicklungs- und vertriebs GmbH
<120> Mittel zur Verbesserung der Immunantwort
<130> XI 1420/02
<150> DE 101 48 697.9
   <151> 2001-10-02
<150> DE 101 56 678.6
   <151> 2001-11-12
<160> 10
<170> PatentIn version 3.1
<210> 1
   <211> 4533
   <212> DNA
   <213> pMOK HBsAg
<220>
   <221> misc_feature
   <222> (1839)..(1045)
   <223> Kanamycin resistance, reverse complementary
<220>
   <221> polyA_site
   <222> (4080)..(4281)
   <223>
<220>
   <221> misc_feature
   <222> (2447)..(3243)
   <223> CMV
<220>
   <221> Intron
   <222> (3250)..(3386)
   <223>
<220>
   <221> misc_feature
   <222> (3393)..(4073)
   <223> Insert HBSAg subtyp ay
<400> 1
   tcttccgctt cctcgctcac tgactcgctg cgctcggtcg ttcggctgcg gcgagcggta      60
   tcagctcact caaaggcggt aatacggtta tccacagaat caggggataa cgcaggaaag     120
   aacatgtctc gggaggcctc acgtgacatg tgagcaaaag gccagcaaaa ggccaggaac     180
   cgtaaaaagg ccgcgttgct ggcgtttttc cataggctcc gcccccctga cgagcatcac     240
   aaaaatcgac gctcaagtca gaggtggcga aacccgacag gactataaag ataccaggcg     300
   tttccccctg gaagctccct cgtgcgctct cctgttccga ccctgccgct taccggatac     360
   ctgtccgcct ttctcccttc gggaagcgtg gcgctttctc atagctcacg ctgtaggtat     420
   ctcagttcgg tgtaggtcgt tcgctccaag ctgggctgtg tgcacgaacc ccccgttcag     480
   cccgaccgct gcgccttatc cggtaactat cgtcttgagt ccaacccggt aagacacgac     540
   ttatcgccac tggcagcagc cactggtaac aggattagca gagcgaggta tgtaggcggt     600
   gctacagagt tcttgaagtg gtggcctaac tacggctaca ctagaaggac agtatttggt     660
   atctgcgctc tgctgaagcc agttaccttc ggaaaaagag ttggtagctc ttgatccggc     720
   aaacaaacca ccgctggtag cggtggtttt tttgtttgca agcagcagat tacgcgcaga     780
   aaaaaaggat ctcaagaaga tcctttgatc ttttctacgg ggtctgacgc tcagtggaac     840
   gaaaactcac gttaagggat tttggtcatg agattatcaa aaaggatctt cacctagatc     900
   cttttaaatt aaaaatgaag ttttaaatca atctaaagta tatatgagta aacttggtct     960
   gacagttacc aatgcttaat cagtgaggca cctatctcag cgatctgtct atttcgttca    1020
   tccatagttg cctgactccc cgtctcagaa gaactcgtca agaaggcgat agaaggcgat    1080
   gcgctgcgaa tcgggagcgg cgataccgta aagcacgagg aagcggtcag cccattcgcc    1140
   gccaagctct tcagcaatat cacgggtagc caacgctatg tcctgatagc ggtccgccac    1200
   acccagccgg ccacagtcga tgaatccaga aaagcggcca ttttccacca tgatattcgg 1260
   caagcaggca tcgccatggg tcacgacgag atcctcgccg tcgggcatgc tcgccttgag 1320
   cctggcgaac agttcggctg gcgcgagccc ctgatgctct tcgtccagat catcctgatc 1380
   gacaagaccg gcttccatcc gagtacgtgc tcgctcgatg cgatgtttcg cttggtggtc 1440
   gaatgggcag gtagccggat caagcgtatg cagccgccgc attgcatcag ccatgatgga 1500
   tactttctcg gcaggagcaa ggtgagatga caggagatcc tgccccggca cttcgcccaa 1560
   tagcagccag tcccttcccg cttcagtgac aacgtcgagc acagctgcgc aaggaacgcc 1620
   cgtcgtggcc agccacgata gccgcgctgc ctcgtcttgc agttcattca gggcaccgga 1680
   caggtcggtc ttgacaaaaa gaaccgggcg cccctgcgct gacagccgga acacggcggc 1740
   atcagagcag ccgattgtct gttgtgccca gtcatagccg aatagcctct ccacccaagc 1800
   ggccggagaa cctgcgtgca atccatcttg ttcaatcata atattattga agcatttatc 1860
   agggttattg tctcatgagc ggatacatat ttgaatgtat ttagaaaaat aaacaaatag 1920
   gggttccgcg cacatttccc cgaaaagtgc cacctgacgt ctaagaaacc attattatca 1980
   tgacattaac ctataaaaat aggcgtatca cgaggccctt tcgtctcgcg cgtttcggtg 2040
   atgacggtga aaacctctga cacatgcagc tcccggagac ggtcacagct tgtctgtaag 2100
   cggatgccgg gagcagacaa gcccgtcagg gcgcgtcagc gggtgttggc gggtgtcggg 2160
   gctggcttaa ctatgcggca tcagagcaga ttgtactgag agtgcaccat atgcggtgtg 2220
   aaataccgca cagatgcgta aggagaaaat accgcatcag gcgccattcg ccattcaggc 2280
   tgcgcaactg ttgggaaggg cgatcggtgc gggcctcttc gctattacgc cagctggcga 2340
   aagggggatg tgctgcaagg cgattaagtt gggtaacgcc agggttttcc cagtcacgac 2400
   gttgtaaaac gacggccagt gccaagcttg gtctcctccc ggatcctcaa tattggccat 2460
   tagccatatt attcattggt tatatagcat aaatcaatat tggctattgg ccattgcata 2520
   cgttgtatct atatcataat atgtacattt atattggctc atgtccaata tgaccgccat 2580
   gttggcattg attattgact agttattaat agtaatcaat tacggggtca ttagttcata 2640
   gcccatatat ggagttccgc gttacataac ttacggtaaa tggcccgcct ggctgaccgc 2700
   ccaacgaccc ccgcccattg acgtcaataa tgacgtatgt tcccatagta acgccaatag 2760
   ggactttcca ttgacgtcaa tgggtggagt atttacggta aactgcccac ttggcagtac 2820
   atcaagtgta tcatatgcca agtccgcccc ctattgacgt caatgacggt aaatggcccg 2880
   cctggcatta tgcccagtac atgaccttac gggactttcc tacttggcag tacatctacg 2940
   tattagtcat cgctattacc atggtgatgc ggttttggca gtacaccaat gggcgtggat 3000
   agcggtttga ctcacgggga tttccaagtc tccaccccat tgacgtcaat gggagtttgt 3060
   tttggcacca aaatcaacgg gactttccaa aatgtcgtaa taaccccgcc ccgttgacgc 3120
   aaatgggcgg taggcgtgta cggtgggagg tctatataag cagaggtcgt ttagtgaacc 3180
   gtcagatcac tagaagcttt attgcggtag tttatcacag ttaaattgct aacgcagtca 3240
   gtgctcgagc aggtaagtat caaggttaca agacaggttt aaggaggcca atagaaactg 3300
   ggcttgtcga gacagagaag actcttgcgt ttctgatagg cacctattgg tcttactgac 3360
   atccactttg cctttctctc cacaggggta ccatggagaa catcacatca ggattcctag    3420
   gaccccttct cgtgttacag gcggggtttt tcttgttgac aagaatcctc acaataccgc    3480
   agagtctaga ctcgtggtgg acttctctca attttctagg gggaactacc gtgtgtcttg 3540
   gccaaaattc gcagtcccca acctccaatc actcaccaac ctcttgtcct ccaacttgtc 3600
   ctggttatcg ctggatgtgt ctgcggcgtt ttatcatctt cctcttcatc ctgctgctat 3660
   gcctcatctt cttgttggtt cttctggact atcaaggtat gttgcccgtt tgtcctctaa 3720
   ttccaggatc ctcaacaacc agcacgggac catgccggac ctgcatgact actgctcaag 3780
   gaacctctat gtatccctcc tgttgctgta ccaaaccttc ggacggaaat tgcacctgta 3840
   ttcccatccc atcatcctgg gctttcggaa aattcctatg ggagtgggcc tcagcccgtt 3900
   tctcctggct cagtttacta gtgccatttg ttcagtggtt cgtagggctt tcccccactg 3960
   tttggctttc agttatatgg atgatgtggt attgggggcc aagtctgtac agcatcttga 4020
   gtcccttttt accgctgtta ccaattttct tttgtctctg ggtatacatt taagagctcg 4080
   atgagtttgg acaaaccaca actagaatgc agtgaaaaaa atgctttatt tgtgaaattt 4140
   gtgatgctat tgctttattt gtaaccatta taagctgcaa taaacaagtt aacaacaaca 4200
   attgcattca ttttatgttt caggttcagg gggaggtgtg ggaggttttt taaagcaagt 4260
   aaaacctcta caaatgtggt agaattcagg gggagaccca attcgtaatc atggtcatag 4320
   ctgtttcctg tgtgaaattg ttatccgctc acaattccac acaacatacg agccggaagc 4380
   ataaagtgta aagcctgggg tgcctaatga gtgagctaac tcacattaat tgcgttgcgc 4440
   tcactgcccg ctttccagtc gggaaacctg tcgtgccagc tgcattaatg aatcggccaa 4500
   cgcgcgggga gaggcggttt gcgtattggg cgc 4533
<210> 2
   <211> 4791
   <212> DNA
   <213> Plasmid pscp36
<220>
   <221> misc_feature
   <222> (1939)..(1045)
   <223> Kanamycin resistance, reverse complementary
<220>
   <221> promoter
   <222> (2447)..(3243)
   <223> CMV
<220>
   <221> Intron
   <222> (3250)..(3386)
   <223>
<220>
   <221> misc_feature
   <222> (3393)..(4331)
   <223> p36 protein
<220>
   <221> polyA_site
   <222> (4338)..(4539)
   <223> poly A site aus sv 40
<400> 2
   tcttccgctt cctcgctcac tgactcgctg cgctcggtcg ttcggctgcg gcgagcggta     60
   tcagctcact caaaggcggt aatacggtta tccacagaat caggggataa cgcaggaaag    120
   aacatgtctc gggaggcctc acgtgacatg tgagcaaaag gccagcaaaa ggccaggaac    180
   cgtaaaaagg ccgcgttgct ggcgtttttc cataggctcc gcccccctga cgagcatcac    240
   aaaaatcgac gctcaagtca gaggtggcga aacccgacag gactataaag ataccaggcg    300
   tttccccctg gaagctccct cgtgcgctct cctgttccga ccctgccgct taccggatac    360
   ctgtccgcct ttctcccttc gggaagcgtg gcgctttctc atagctcacg ctgtaggtat    420
   ctcagttcgg tgtaggtcgt tcgctccaag ctgggctgtg tgcacgaacc ccccgttcag    480
   cccgaccgct gcgccttatc cggtaactat cgtcttgagt ccaacccggt aagacacgac    540
   ttatcgccac tggcagcagc cactggtaac aggattagca gagcgaggta tgtaggcggt    600
   gctacagagt tcttgaagtg gtggcctaac tacggctaca ctagaaggac agtatttggt    660
   atctgcgctc tgctgaagcc agttaccttc ggaaaaagag ttggtagctc ttgatccggc    720
   aaacaaacca ccgctggtag cggtggtttt tttgtttgca agcagcagat tacgcgcaga    780
   aaaaaaggat ctcaagaaga tcctttgatc ttttctacgg ggtctgacgc tcagtggaac    840
   gaaaactcac gttaagggat tttggtcatg agattatcaa aaaggatctt cacctagatc    900
   cttttaaatt aaaaatgaag ttttaaatca atctaaagta tatatgagta aacttggtct 960
   gacagttacc aatgcttaat cagtgaggca cctatctcag cgatctgtct atttcgttca 1020
   tccatagttg cctgactccc cgtctcagaa gaactcgtca agaaggcgat agaaggcgat 1080
   gcgctgcgaa tcgggagcgg cgataccgta aagcacgagg aagcggtcag cccattcgcc 1140
   gccaagctct tcagcaatat cacgggtagc caacgctatg tcctgatagc ggtccgccac 1200
   acccagccgg ccacagtcga tgaatccaga aaagcggcca ttttccacca tgatattcgg 1260
   caagcaggca tcgccatggg tcacgacgag atcctcgccg tcgggcatgc tcgccttgag 1320
   cctggcgaac agttcggctg gcgcgagccc ctgatgctct tcgtccagat catcctgatc 1380
   gacaagaccg gcttccatcc gagtacgtgc tcgctcgatg cgatgtttcg cttggtggtc 1440
   gaatgggcag gtagccggat caagcgtatg cagccgccgc attgcatcag ccatgatgga 1500
   tactttctcg gcaggagcaa ggtgagatga caggagatcc tgccccggca cttcgcccaa 1560
   tagcagccag tcccttcccg cttcagtgac aacgtcgagc acagctgcgc aaggaacgcc 1620
   cgtcgtggcc agccacgata gccgcgctgc ctcgtcttgc agttcattca gggcaccgga 1680
   caggtcggtc ttgacaaaaa gaaccgggcg cccctgcgct gacagccgga acacggcggc 1740
   atcagagcag ccgattgtct gttgtgccca gtcatagccg aatagcctct ccacccaagc 1800
   ggccggagaa cctgcgtgca atccatcttg ttcaatcata atattattga agcatttatc 1860
   agggttattg tctcatgagc ggatacatat ttgaatgtat ttagaaaaat aaacaaatag 1920
   gggttccgcg cacatttccc cgaaaagtgc cacctgacgt ctaagaaacc attattatca 1980
   tgacattaac ctataaaaat aggcgtatca cgaggccctt tcgtctcgcg cgtttcggtg 2040
   atgacggtga aaacctctga cacatgcagc tcccggagac ggtcacagct tgtctgtaag 2100
   cggatgccgg gagcagacaa gcccgtcagg gcgcgtcagc gggtgttggc gggtgtcggg 2160
   gctggcttaa ctatgcggca tcagagcaga ttgtactgag agtgcaccat atgcggtgtg 2220
   aaataccgca cagatgcgta aggagaaaat accgcatcag gcgccattcg ccattcaggc 2280
   tgcgcaactg ttgggaaggg cgatcggtgc gggcctcttc gctattacgc cagctggcga 2340
   aagggggatg tgctgcaagg cgattaagtt gggtaacgcc agggttttcc cagtcacgac 2400
   gttgtaaaac gacggccagt gccaagcttg gtctcctccc ggatcctcaa tattggccat 2460
   tagccatatt attcattggt tatatagcat aaatcaatat tggctattgg ccattgcata 2520
   cgttgtatct atatcataat atgtacattt atattggctc atgtccaata tgaccgccat 2580
   gttggcattg attattgact agttattaat agtaatcaat tacggggtca ttagttcata 2640
   gcccatatat ggagttccgc gttacataac ttacggtaaa tggcccgcct ggctgaccgc 2700
   ccaacgaccc ccgcccattg acgtcaataa tgacgtatgt tcccatagta acgccaatag 2760
   ggactttcca ttgacgtcaa tgggtggagt atttacggta aactgcccac ttggcagtac 2820
   atcaagtgta tcatatgcca agtccgcccc ctattgacgt caatgacggt aaatggcccg 2880
   cctggcatta tgcccagtac atgaccttac gggactttcc tacttggcag tacatctacg 2940
   tattagtcat cgctattacc atggtgatgc ggttttggca gtacaccaat gggcgtggat 3000
   agcggtttga ctcacgggga tttccaagtc tccaccccat tgacgtcaat gggagtttgt 3060
   tttggcacca aaatcaacgg gactttccaa aatgtcgtaa taaccccgcc ccgttgacgc 3120
   aaatgggcgg taggcgtgta cggtgggagg tctatataag cagaggtcgt ttagtgaacc 3180
   gtcagatcac tagaagcttt attgcggtag tttatcacag ttaaattgct aacgcagtca 3240
   gtgctcgagc aggtaagtat caaggttaca agacaggttt aaggaggcca atagaaactg 3300
   ggcttgtcga gacagagaag actcttgcgt ttctgatagg cacctattgg tcttactgac 3360
   atccactttg cctttctctc cacaggggta ccatgaacta cgagggtcac ctgaagggcc 3420
   accgcggatg ggtcacctcc ctggcctgcc cgcagcaggc ggggtcgtac atcaaggtgg 3480
   tgtcgacgtc gcgcgatggc acggccatct cgtggaaagc caaccccgac cgccacagcg 3540
   tggacagcga ctacggtctg ccgagccacc gcctcgaggg ccacaccggc ttcgtgtcgt 3600
   gtgtgtcgct ggcccacgcc accgactacg cgctgaccgc gtcctgggac cgctccatcc 3660
   gcatgtggga cctgcgcaat ggccagtgcc agcgcaagtt cctgaagcac accaaggacg 3720
   tgctcgccgt cgccttctcg ccggacgacc gcctgatcgt gtccgcgggc cgcgacaacg 3780
   tgatccgcgt gtggaacgtg gcgggcgagt gcatgcacga gttcctgcgc gacggccacg 3840
   aggactgggt gagcagcatc tgtttctcgc cgtcgctgga gcatccgatc gtggtgtccg 3900
   gcagctggga caacaccatc aaggtatgga acgtgaacgg gggcaagtgt gagcgcacgc 3960
   tcaagggcca cagcaactac gtgtccacgg tgacggtgtc gccagacggg tcgctgtgcg 4020
   cgtccggcgg caaggacggc gcggcgctgc tgtgggacct gagcaccggc gagcagctgt 4080
   tcaagatcaa cgtggagtcg cccatcaacc agatcgcctt ctcgcccaac cgcttctgga 4140
   tgtgcgtcgc gacggagagg tccctgtccg tgtacgacct ggagagcaag gctgtgattg 4200
   cggagctgac gccggacggc gcgaagccgt ccgagtgcat ctccattgcc tggtccgccg 4260
   acggcaacac tctgtactcc ggtcacaagg acaacctgat ccgcgtgtgg tccatctccg 4320
   acgccgagta agagctcgat gagtttggac aaaccacaac tagaatgcag tgaaaaaaat 4380
   gctttatttg tgaaatttgt gatgctattg ctttatttgt aaccattata agctgcaata 4440
   aacaagttaa caacaacaat tgcattcatt ttatgtttca ggttcagggg gaggtgtggg 4500
   aggtttttta aagcaagtaa aacctctaca aatgtggtag aattcagggg gagacccaat 4560
   tcgtaatcat ggtcatagct gtttcctgtg tgaaattgtt atccgctcac aattccacac 4620
   aacatacgag ccggaagcat aaagtgtaaa gcctggggtg cctaatgagt gagctaactc 4680
   acattaattg cgttgcgctc actgcccgct ttccagtcgg gaaacctgtc gtgccagctg 4740
   cattaatgaa tcggccaacg cgcggggaga ggcggtttgc gtattgggcg c 4791
<210> 3
<211> 11
<212> PRT
<213> Human immunodeficiency virus type 1, TAT peptide
<400> 3
   Tyr Gly Arg Lys Lys Arg Arg Gln Arg Arg Arg
   1 5 10
<210> 4
   <211> 7
   <212> PRT
   <213> simian virus 40, NLS peptide
<400> 4
   Pro Lys Lys Lys Arg Lys Val
   1 5
<210> 5
   <211> 20
   <212> DNA
   <213> ODN 1
<220>
   <221> modified_base
   <222> (12)..(12)
   <223> xT = Thymin modified with a reactive amino group
<400> 5
   gggagtccag ttttctggac 20
<210> 6
   <211> 20
   <212> DNA
   <213> ODN 2
<400> 6
   aggggtccag ttttctggac 20
<210> 7
   <211> 33
   <212> DNA
   <213> 1. PCR: Primer left
<400> 7
   ttatatggta ccatgaacat acgagggtca cct 33
<210> 8
   <211> 42
   <212> DNA
   <213> 1. PCR: Primer right
<400> 8
   ttatatgagc tcagaagaca cggacaggga cctcttccgt cg 42
<210> 9
   <211> 33
   <212> DNA
   <213> 2. PCR: Primer left
<400> 9
   ttatatggta ccatgaacat acgagggtca cct 33
<210> 10
   <211> 33
   <212> DNA
   <213> 2. PCR: Primer right
<400> 10
   ttatatgagc tcttactcgg ccgtcggaga tgg 33